# EUROPEAN PATENT SPECIFICATION

(11) **EP 1 299 313 B1**
(45) Date of publication and mention of the grant of the patent: **21.04.2004**
(21) Application number: 01969373.8
(22) Date of filing: 12.07.2001
(51) Int. Cl.: C02F 1/78, A61K 33/06, C01F 5/30

(54) **PROCESS FOR THE DEBROMINATION OF AN AQUEOUS SALT SOLUTION USING OZONE**
VERFAHREN ZUR DEBROMINIERUNG VON WÄSSRIGEN SALZLÖSUNGEN DURCH OZON
PROCESSUS DESTINE A LA DEBROMATION D'UNE SOLUTION AQUEUSE DE SEL AU MOYEN D'OZONE

(30) Priority: 12.07.2000 DE 10033762
(43) Date of publication of application: 09.04.2003
(73) Proprietor: Honeywell Specialty Chemicals Seelze GmbH, 30926 Seelze (DE)
(72) Inventor: KLINGENBERG, Andreas, 30827 Garbsen (DE); KLASS, Manfred, 21684 Stade (DE)
(74) Representative: Geissler, Bernhard, Dr. jur., Dipl.-Phys.
(86) International application number: PCT/EP2001/008063
(87) International publication number: WO 2002/004363

(56) References cited:
- DE-A- 4 428 147
- DE-A- 19 830 310
- US-A- 5 980 854
- US-A- 6 024 882

## Description

The present invention relates to a process for the debromination of a salt solution containing impurities of bromide by the use of ozone.

In many areas of application, as e.g. chemistry or pharmacy, the maximum bromide content of the chemical compounds employed is problematic. The maintenance of upper limits concerning the bromide concentration is *inter alia* important in the field of pharmaceutical industry.

If bromide-containing chemicals are used in the pharmaceutical area, the purity requirements of the relevant pharmacopoeiae (Pharma Euro III, p. 1234-1235) are taken as a basis. Compounds which are important in the present context are, for example, alkali and earth alkali compounds.

In particular, in alkali and earth alkali compounds which are of natural origin, the bromide content present therein embodies an important problem, because numerous anions of e.g. alkali and earth alkali salts are present in the company of bromide. The latter, for example, holds true for naturally-occurring alkali and earth alkali chlorides.

The European Pharmaceutical Codex since January 1, 1997, requires for magnesium chloride, for example, a concentration limit concerning the bromide content of 500 ppm.

A process for the reduction of the bromide content by means of which the above-mentioned all-over bromide content of a compound can be achieved, is, for example, embodied by the introduction of gaseous chlorine into a solution of the particular compound present in a suitable solvent, as for example water.

The publication DE-A 21 18 623 discloses a process for the purification of aqueous magnesium chloride solutions in which the bromide content is reduced by introducing chlorine gas into these solutions.

In the publication DE-A 26 13 288 a process for the manufacture of highly concentrated magnesium chloride solutions is described. The debromination described in the latter document is accomplished by means of gaseous chlorine in the presence of heat.

The handling of gaseous chlorine, however, embodies a high potential of danger because of the reactivity of the latter gas. Accordingly, for practicing the particular processes or, respectively, experimental setups, in general for safety reasons a highly sophisticated choice of apparatuses and, therefore, also a substantial financial effort is required.

The document WO-A-00/02815 (PCT/EP99/04515) describes a method for the bromide depletion of an aqueous solution by using hydrogen peroxide which accomplishes the reduction of the number of required process steps.

However, the latter process requires a substantial acidification of the solution, which on the one hand enhances the corrosive properties of the solution, and on the other hands renders the reduction of the excess acid in order to maintain a predetermined pH value necessary thereby diminishing the economical feasibility of the process by adding the above-mentioned additional process step.

Therefore, it was an objective of the present invention to provide a technical process which can be conducted using simply apparatuses, in which solutions can be depleted of bromide without having to dilute or acidify the solution.

This objective is solved by the use of ozone as means of oxidation for bromide.

Accordingly, the present invention relates to a process for the debromination of a concentrated aqueous alkali metal chloride and/or earth alkali metal chloride solution containing impurities of bromide, in which the solution is contacted with ozone and the bromine thus formed, as well as excess ozone, are separated from the,aqueous solution.

In particular, the present invention relates to a process characterized in that concentrated aqueous solution contains the alkali metal and/or earth alkali metal chloride in an amount of preferred > 15 wt.-%, more preferred > 30 wt.-%, even more preferred > 50 wt.-%, most preferred > 65 wt.-%, with crystalline chloride salt taken as a basis for the calculation.

Preferred the alkali metal and/or earth alkali metal chloride content as used in the process according to the invention is > 25 wt.-%, more preferred > 30 wt.-%, even more preferred > 50 wt.-% and particularly > 65 wt.-%, respectively calculated as crystalline chloride salt.

Among the most important compounds, the solutions of which are treated by the process according to the invention in order to achieve debromination, are concentrated aqueous magnesium chloride (MgCl₂-6-H₂0) solutions which serve as basic solutions for the crystallization of MgCl₂-6-H₂O according to Ph. Eur. 3^{rd}, which allows for a bromine content in the crystalline body which amounts to a maximum of 500 ppm.

Accordingly, the present invention also relates to a process characterized in that the concentrated aqueous solution is a concentrated aqueous magnesium chloride solution.

The content of one or more bromide impurity/impurities in the concentrated aqueous magnesium chloride solution lies in the range of > 100 ppm, more preferred in the range of > 1.000 ppm, and most preferred in the range of 1.000 to 4.000 ppm, in relation to solid MgCl₂-6-H₂O.

Such solutions are obtained as highly concentrated magnesium chloride nobel brines containing bromide impurities from the processing of carnallitic kali raw salts, with bromine being in part found in the crystalline body upon crystallization, rendering the latter of restricted usefulness for the manufacture of MgCl₂-6-H₂O.

Therefore, the present invention also relates to a process which is characterized in that the concentrated aqueous solution is a concentrated aqueous solution of MgCl₂-6-H₂0 having a content of one or more bromide impurity/impurities derived from the processing of carnallitic kali raw salts.

The ozone is produced from oxygen *in situ* using an ozone generating device with the oxygen or air enriched in oxygen being passed through the concentrated aqueous solution in a mixture with ozone.

In general, it is not critical in the process according to the invention, in which concentration ozone is employed for the oxidation of bromide to bromine. According to the invention, the concentration of ozone in the gaseous oxygen or in the air enriched in gaseous oxygen lies in the range of 1 to 25 vol.-%, preferred in the range of 5 to 15 vol.-%. The admission of oxygen or air enriched in oxygen to the solution amounts to 103 l/h or 51.6 l/h, respectively, for a laboratory scale setup, with an industrial scale setup being possibly designed for e.g. 1 Nm³/h.

The mass ratio of absorbed ozone to oxidized bromide desirably lies in the range of about 0.2 to 0.6 : 1, which corresponds to a molar ratio of about 0.3 to 0.75 : 1.

In the concentration range examined, the process does not exhibit any pH dependency; the reaction kinetics do not show any acid catalysis. The pH value of the starting solution in the process according to the invention generally lies in the range of 6 ± 1, which corresponds to a pH value of 8 to 9 at a dilution of 1 to 10.

Using the process according to the invention, after the removal of elementary bromine, magnesium chloride is obtained which has a bromide content being < 500 ppm, preferred < 250 ppm, in relation to crystalline magnesium chloride. Upon longer lasting turnover of the alkali metal and/or earth alkali metal solution containing impurities of bromide with ozone and the removal of elementary bromine, magnesium chloride is obtained in which bromide is not detectable any more.

The temperature at which the process according to the invention is carried out lies usually in the range of about 0°C to 80°C, preferred in the range of 25 ± 10°C.

A further advantage of the process according to the invention is embodied by the fact that bromate eventually formed may undergo comproportionation with bromide to bromine which is removed according to the invention.

The bromine formed by oxidation during the course of the process may be removed from the solution according to all processes known in the state of the art. In particular, the bromine formed during the debromination process by oxidation using ozone is expelled together with excess ozone using the stream of oxygen gas or the air enriched in oxygen gas. The volume stream is highly dependent on the scale of the plant. Oxygen or air enriched in oxygen is blown into the solution with a turnover of generally 10 to 200 l/h, preferred 50 to 150 l/h (laboratory experiment); 0.1 to 10 m³/h, preferred 1 to 5 m³/h (6.000 l reactor); and 1 to 50 m³/h, preferred 5 to 20 m³/h (40.000 l reactor). The bromine expelled by the exhaust gas is washed out in an exhaust gas washing device, which, for example, may be run using a thiosulfate solution.

Furthermore, the present invention in its most general embodiment relates to the use of ozone in a process for the debromination of a concentrated aqueous alkali metal and/or earth alkali metal chloride solution containing impurities of bromide, in particular magnesium chloride solution, in which the solution is contacted with ozone and bromine thus formed as well as excess ozone, is removed from the aqueous solution.

Further, the present invention relates to the use of magnesium chloride having a bromide content of < 250 ppm, in relation to crystalline magnesium chloride, which is manufactured according to the process according to the invention by subsequent crystallization of the magnesium chloride solution, to be used for medical purposes. The highly pure magnesium chloride is preferred employed for the manufacture of hemodialysis, hemofiltration or peritoneal dialysis solutions and for parenteral applications, in particular for infusion solutions. Thus, the present invention also relates to the use of magnesium chloride as specified above for the production of pharmaceuticals, particularly for the production of an aqueous solution for dialysis purposes.

The present invention will be explained hereinafter by means of worked examples.

### Examples

In a technical process, 14 l of a MgCl₂ brine, the percentage of which, calculated as MgCl₂-6-H₂O, was between 72.8 % and 73.7 % and which contained 1.200 to 1.300 ppm bromide (in relation to the solid), were added to a batch reactor equipped with a stirrer using radial addition of ozone gas in a gas stream of 103 l/h (ozone concentration amounting to about 13 %) or of 51.6 l/h (ozone concentration amounting to about 14.5 %). The pH value of the starting solution was in the range of 5 to 6, which corresponded to a pH value of 8 to 10 at a dilution of 1 to 10. The pH value of the solution was recorded on-line. The remaining bromide content of the solution was determined in time intervals of 5 or 15 minutes, respectively, as well as the ozone content in the feed gas stream and the off gas stream. Furthermore, the bromate content of the debrominated solution was determined by ion chromatography.

It was observed that the solution was turning yellowish already after 5 minutes of ozone addition. The yellow color turned more intense after 30 minutes of ozone addition and from that point on remained constant.

From the table provided hereinbelow, the values for the remaining bromide content of the starting solutions obtained by modifying the reaction conditions can be seen. In all three experiments, the bromide content after 25 or 35 minutes of reaction time, respectively, was at < 500 ppm or, respectively, bromide was not any more detectable in the starting solution after 45 minutes or 55 minutes, respectively, of reaction time.

| Parameter | Example 1 | Example 2 | Example 3 |
|---|---|---|---|
| free acid (HCl) calculated as solid content | 0.005 % | --- | --- |
| free alkali (NaOH) calculated as solid content | --- | 0.008 % | 0.008 % |
| amount of gas/ ozone content | 103 l/h 13 % | 103 l/h 13 % | 51.6 l/h 14.5 % |
| bromide content of the starting solution (calculated as solid content in relation to MgCl₂-6-H₂O) | 0.13 % | 0.13 % | 0.13 % |
| reaction time (bromide < 500 ppm) | 25 min. | 25 min. | 35 min. |
| reaction time (bromide not detectable) | 45 min. | 55 min. | 45 min. |
| absorption efficiency | 48 % | 45 % | 60 % |
| fraction ozone:bromide (mass) | 0.4 : 1 | 0.45 : 1 | 0.27 : 1 |
| fraction ozone:bromide (moles) | 0.6 : 1 | 0.76 : 1 | 0.45 : 1 |

## Claims

1. Process for the debromination of a concentrated aqueous alkali metal chloride and/or earth alkali metal chloride solution containing impurities of bromide, wherein the solution is contacted with ozone and bromine thus formed as well as excess ozone is removed from the aqueous solution.

2. Process according to claim 1, in which the concentrated aqueous solution contains the alkali metal and/or earth alkali metal chloride in an amount of > 15 wt.-%, calculated as crystalline chloride salt.

3. Process according to claim 2, in which the alkali metal and/or earth alkali metal chloride content is > 25 wt.-%, calculated as crystalline chloride salt.

4. Process according to claim 3, in which the alkali metal and/or earth alkali metal chloride content is > 30 wt.-%, calculated as crystalline chloride salt.

5. Process according to one or several of claims 1 to 4, in which the concentrated aqueous solution is a concentrated aqueous magnesium chloride solution.

6. Process according to claim 5, in which the concentrated aqueous solution is a concentrated aqueous magnesium chloride solution, wherein the amount of one or more bromide impurity/impurities is in the range of > 100 ppm, in relation to solid MgCl₂-6-H₂0.

7. Process according to claim 6, in which the concentrated aqueous solution is a concentrated aqueous magnesium chloride solution, wherein the amount of the one or more bromide impurity/impurities is in the range of > 1.000 ppm, in relation to solid MgCl₂-6-H₂0.

8. Process according to claim 7, in which the concentrated aqueous solution is a concentrated aqueous magnesium chloride solution, wherein the amount of one or more bromide impurity/impurities is in the range of 1.000 to 4.000 ppm, in relation to solid MgCl₂-6-H₂0.

9. Process according to one or several of claims 1 to 8, in which the concentrated aqueous solution is a concentrated aqueous solution of MgCl₂-6-H₂0 having a content concerning one or more bromide impurity/impurities derived from the processing of carnallitic kali raw salts.

10. Process according to one or several of claims 1 to 9, in which ozone is generated *in situ* from oxygen.

11. Process according to claim 10, in which ozone, which is *in situ* generated in the mixture with oxygen is passed through the concentrated aqueous solution in the form of oxygen gas or air enriched in oxygen gas.

12. Process according to claim 11, in which the concentration of ozone in the oxygen gas or in the air enriched in oxygen gas is in the range of 1 to 25 vol.-%, preferred in the range of 5 to 15 vol.-%.

13. Process according to one or several of claims 1 to 12, in which the pH value is in the range of 6 ± 1.

14. Process according to one or several of claims 1 to 13, in which magnesium chloride is manufactured having a Br⁻-content of < 500 ppm, in relation to crystalline MgCl₂-6-H₂O.

15. Process according to claim 14, in which magnesium chloride is manufactured having a Br⁻-content of < 250 ppm, in relation to crystalline MgCl₂-6-H₂O.

16. Process according to one or several of claims 1 to 15, which is conducted at a temperature of 0°C to 80°C, preferred 25 ± 10°C.

17. Process according to one or several of claims 1 to 16, in which the bromine formed by the oxidation of bromide using ozone is expelled from the concentrated aqueous solution together with excess ozone in the oxygen stream or air / oxygen stream, respectively, and optionally is washed out in a gas washer.

18. Use of ozone for the debromination of a concentrated aqueous alkali metal chloride and/or earth alkali metal chloride solution, in particular magnesium chloride solution, containing one or more bromide impurities being used in the manufacture of pharmaceuticals.

## Patentansprüche

1. Verfahren zum Entbromen einer konzentrierten wässrigen, Bromid-Verunreinigung enthaltenden Alkalimetall- und/oder Erdalkalimetallchlorid-Lösung, worin man die Lösung mit Ozon in Kontakt bring und entstandenes Brom sowie überschüssiges Ozon aus der wässrigen Lösung abtrennt.

2. Verfahren nach Anspruch 1, worin der Gehalt der konzentrierten wässrigen Lösung an Alkalimetall- und/oder Erdalkalimetallchlorid > 30 Gew.-% beträgt, berechnet als kristallines Chloridsalz.

3. Verfahren nach Anspruch 2, worin der Gehalt an Alkalimetall- und/oder Erdalkalimetallchlorid > 50 Gew.-% beträgt, berechnet als kristallines Chloridsalz.

4. Verfahren nach Anspruch 3, worin der Gehalt an Alkalimetall- und/oder Erdalkalimetallchlorid > 65 Gew.-% beträgt, berechnet als kristallines Chloridsalz.

5. Verfahren nach einem oder mehreren Ansprüchen 1 bis 4, worin die konzentrierte wässrige Lösung eine konzentrierte wässrige Magnesiumchloridlösung ist.

6. Verfahren nach Anspruch 5, worin die konzentrierte wässrige Lösung eine konzentrierte wässrige Magnesiumchlorid-Lösung mit einem Gehalt an einer oder mehreren Bromid-Verunreinigung(en) im Bereich von > 100 ppm, ist bezogen auf festes MgCl₂.

7. Verfahren nach Anspruch 6, worin die konzentrierte wässrige Lösung eine konzentrierte wässrige Magnesiumchlorid-Lösung mit einem Gehalt an einer oder mehreren Bromid-Verunreinigung(en) im Bereich von > 1.000 ppm ist, bezogen auf festes MgCl₂.

8. Verfahren nach Anspruch 7, worin die konzentrierte wässrige Lösung eine konzentrierte wässrige Magnesiumchlorid-Lösung mit einem Gehalt an einer oder mehreren Bromid-Verunreinigung(en) im Bereich von 1.000 bis 4.000 ppm ist, bezogen auf festes MgCl₂.

9. Verfahren nach einem oder mehreren der Ansprüche 1 bis 8, worin die wässrige Lösung eine konzentrierte wässrige Lösung von MgC12 mit einem Gehalt an einer oder mehreren Bromid-Verunreinigung(en) aus der Verarbeitung carnallitischer Kalirohsalze ist.

10. Verfahren nach einem oder mehreren der Ansprüche 1 bis 9, worin das Ozon in situ aus Sauerstoff erzeugt wird.

11. Verfahren nach Anspruch 10, worin das Ozon in situ aus Sauerstoff erzeugt wird, der durch die konzentrierte wässrige Lösung geleitet wird, bevorzugt in situ aus Sauerstoff erzeugt wird, der in Form von Sauerstoffgas oder mit Sauerstoffgas angereicherter Luft durch die konzentrierte wässrige Lösung geleitet wird.

12. Verfahren nach Anspruch 11, worin die Konzentration des Ozons in dem Sauerstoffgas oder in der mit Sauerstoffgas angereicherten Luft im Bereich von 1 bis 25 Vol.-% liegt, bevorzugt im Bereich von 5 bis 15 Vol.-%.

13. Verfahren nach einem oder mehreren der Ansprüche 1 bis 12, worin der pH-Wert im Bereich von 6 ± 1 liegt.

14. Verfahren nach einem oder mehreren der Ansprüche 1 bis 13, worin Magnesiumchlorid mit einem Br-Gehalt von < 500 ppm hergestellt wird, bezogen auf kristallines Magnesiumchlorid.

15. Verfahren nach Anspruch 14, worin Magnesiumchlorid mit einem Br-Gehalt von < 250 ppm, hergestellt wird, bezogen auf kristallines Magnesiumchlorid.

16. Verfahren nach einem oder mehreren der Ansprüche 1 bis 15, welches bei 25 °C ± 10 °C durchgeführt wird.

17. Verfahren nach einem oder mehreren der Ansprüche 1 bis 16, worin das im Rahmen der Oxidation von Bromid mit Ozon entstandene Brom zusammen mit überschüssigem Ozon mit dem Sauerstoffstrom bzw. Luft/Sauerstoffstrom aus der konzentrierten wässrigen Lösung ausgetrieben und gegebenenfalls in einem Abgaswäscher ausgewaschen wird.

18. Verwendung von Ozon zum Entbromen einer konzentrierten wässrigen, eine oder mehrere Bromid-Verunreinigung(en) enthaltenden Alkalimetall und/oder Erdalkalimetallchlorid-Lösung, insbesondere Magnesiumchlorid-Lösung, gemäß dem Verfahren nach Anspruch 1, die bei der Herstellung von Arzneimittel verwendet wird.

## Revendications

1. Procédé pour la débromation d'une solution de chlorure de métal alcalino-terreux et/ou de chlorure de métal alcalin aqueuse renfermant des impuretés de brome, dans lequel la solution est mise en contact avec de l'ozone et le brome ainsi formé ainsi que l'ozone en excès sont éliminés de la solution aqueuse.

2. Procédé selon la revendication 1, dans lequel la solution aqueuse concentrée renferme le chlorure de métal alcalino-terreux et/ou de métal alcalin selon une quantité supérieure à 15 % en poids, calculé en tant que chlorure cristallin.

3. Procédé selon la revendication 2, dans lequel la teneur en chlorure de métal alcalino-terreux et/ou alcalin est supérieure à 25 % en poids, calculé en tant que chlorure cristallin.

4. Procédé selon la revendication 3, dans lequel la teneur en chlorure de métal alcalino-terreux et/ou en métal alcalin est supérieure à 30 % en poids, calculé en tant que chlorure cristallin.

5. Procédé selon l'une ou plusieurs des revendications 1 à 4, dans lequel la solution aqueuse concentrée est une solution de chlorure de magnésium aqueuse concentrée.

6. Procédé selon la revendication 5, dans lequel la solution aqueuse concentrée est une solution de chlorure de magnésium aqueuse concentrée, dans lequel la quantité d'une ou plusieurs impureté/impuretés de brome se situe dans une gamme supérieure à 100 ppm par rapport au MgCl₂-6-H₂O solide.

7. Procédé selon la revendication 6, dans lequel la solution aqueuse concentrée est une solution de chlorure de magnésium aqueuse concentrée, dans lequel la quantité d'une ou plusieurs impureté/impuretés de brome se situe dans une gamme supérieure à 1 000 ppm par rapport au MgCl₂-6-H₂O solide.

8. Procédé selon la revendication 7, dans lequel la solution aqueuse concentrée est une solution de chlorure de magnésium aqueuse concentrée, dans lequel la quantité d'une ou plusieurs impureté/impuretés de brome se situe dans la gamme de 1 000 à 4 000 ppm par rapport au MgCl₂-6-H₂O solide.

9. Procédé selon l'une ou plusieurs des revendications 1 à 8, dans lequel la solution aqueuse concentrée est une solution aqueuse concentrée de MgCl₂-6-H₂O ayant une teneur renfermant une ou plusieurs impureté/impuretés de brome dérivées du procédé de traitement de sels bruts de kali carnallitique.

10. Procédé selon l'une ou plusieurs des revendications 1 à 9, dans lequel l'ozone est généré in situ à partir d'oxygène.

11. Procédé selon la revendication 10, dans lequel l'ozone, qui est généré in situ dans le mélange avec de l'oxygène, traverse la solution aqueuse concentrée sous la forme de gaz oxygène ou d'air enrichi en gaz oxygène.

12. Procédé selon la revendication 11, dans lequel la concentration d'ozone dans le gaz oxygène ou dans l'air enrichi en gaz oxygène se situe dans la gamme de 1 à 25 % en volume, de préférence dans la gamme de 5 à 15 % en volume.

13. Procédé selon l'une ou plusieurs des revendications 1 à 12, dans lequel le pH se situe dans la gamme de 6 ± 1.

14. Procédé selon l'une ou plusieurs des revendications 1 à 13, dans lequel le chlorure de magnésium est fabriqué en ayant une teneur en Br⁻ inférieure à 500 ppm par rapport au MgCl₂-6-H₂O cristallin.

15. Procédé selon la revendication 14, dans lequel le chlorure de magnésium est fabriqué en ayant une teneur en Br⁻ inférieure à 250 ppm par rapport au MgCl₂-6-H₂O cristallin.

16. Procédé selon l'une ou plusieurs des revendications 1 à 15, qui est mis en oeuvre à une température de 0° C à 80° C, de préférence de 25° C ± 10° C.

17. Procédé selon l'une ou plusieurs des revendications 1 à 16, dans lequel le brome formé par l'oxydation du brome en utilisant de l'ozone est expulsé de la solution aqueuse concentrée conjointement avec l'ozone en excès dans le courant d'oxygène ou le courant d'air/oxygène respectivement et est éventuellement séparé par lavage dans un dispositif de lavage de gaz.

18. Utilisation de l'ozone pour la débromation. d'une solution de chlorure de métal alcalino-terreux et/ou de chlorure de métal alcalin aqueuse, en particulier une solution de chlorure de magnésium renfermant une ou plusieurs impuretés de brome utilisée dans la fabrication de produits pharmaceutiques.
